# EUROPEAN PATENT APPLICATION

(11) **EP 1 146 120 A1**
(43) Date of publication of application: **17.10.2001**
(21) Application number: 01400922.9
(22) Date of filing: 10.04.2001
(51) Int. Cl.: C12N 5/08, G01N 33/50, C07K 14/045

(54) **Method for obtaining specific T-lymphocites, and for identifying etitopes**

(30) Priority: 12.04.2000 EP 00401019
(71) Applicant: INSTITUT NATIONAL DE LA SANTE ET DE LA RECHERCHE MEDICALE (INSERM), 75654 Paris Cédex 13 (FR)
(72) Inventor: Vie, Henri, 4400 nantes (FR); Ibisch, Catherine, 4400 Nantes (FR)
(74) Representative: Becker, Philippe

(57) **Abstract**

The present invention relates to a method for obtaining T-lymphocytes specific for known or unknown epitopes, and for further identifying said epitopes if needed. This invention also includes methods of ex vivo or in vitro production of antigen-specific T cells, as well as compositions and methods for regulating an immune response in a subject. Preferred compositions comprise T cells specific for viral or tumor antigens and can be used to regulate an immune response against viral infection or tumor development or progression in a subject.

## Description

The present invention relates to a method for obtaining T-lymphocytes specific for known or unknown epitopes, and for further identifying said epitopes if needed. This invention also includes methods of ex vivo or in vitro production of antigen-specific T cells, as well as compositions and methods for regulating an immune response in a subject. Preferred compositions comprise T cells specific for viral or tumor antigens and can be used to regulate an immune response against viral infection or tumor development or progression in a subject.

Improvement of selection methods for efficient recovery of human specific T-lymphocytes has general implication for immunologists. First, it can help to define the antigens expressed by pathogens and tumors that are recognized by T cells and second it can facilitate the preparation of specific T-cells lines for adoptive immunotherapy. Indeed, adoptive cell therapy with specific cytotoxic T-lymphocytes now appears as a promising approach, particularly in the cas of Epstein-Barr virus (EBV) or cytomegalovirus (CMV) infections affecting immunocompromised patients (Heslop et al (1994); Walter et al, (1995)).

From an immunological point of view, accumulating data in the literature tend to favor the notion that immune response against these two viruses is essentially focused against a few proteins. For example frequent responses against the peptide GLCTLVAML (SEQ ID NO: 11) from the EBV-early lytic protein BMLF1 and frequent responses against the peptide NLVPMVATV (SEQ ID NO: 10) from the CMV-enveloppe phosphoprotein pp65 were observed among peripheral blood mononuclear cells of seropositive HLA-A*0201 individuals (Steven et al (1997); Wills et al (1996)).

From a clinical point of view essentially two constrains limit a broader usage of T cell therapy against viruses: the delay required to obtain specific T-cells and the safety of the selection procedure. In term of delay, selection of EBV-specific T-cells requires the generation of an autologous B lymphoblastoid cell line (LCL) as EBV antigen presenting cells followed by a coculture together with autologous PBMC. Several weeks are required to obtain the LCL and several other weeks in addition to enrich for EBV-specific T cells (Heslop H et al (1994)). In term of safety, this procedure uses biological material from two different xenogenic origin: simian for the cell line and bovine for the serum, (LCL are obtained after infection of autologous PBMC with an EBV viral strain produced by the marmoset B95.8 cell line which is cultured in the presence of fetal calf serum). A comparable level of complexity is associated with the preparation of CMV-specific T-lymphocytes. Obviously, a significant progress would consist in eliminating the need for antigen-presenting cells preparation, or the need of an infectious viral strain, for safety concern. The possibility to reduce the length of cell cultures in vitro as well as the ability to increase the selectivity of T cells would provide significant value and allow the broad use of the technology in various clinical conditions.

However, the current tendency of the scientists is still to search for the most efficient antigen-presenting cells and for instance many authors work on the purification of activated dendritic or B cells (Borst et al, 1999). Furthermore, while several selection methods have been proposed, Such as an immunomagnetic selection based on the production of interferon gamma by specific T-lymphocytes (Brosterhus et al, (1999)), none is easily applicable to generate efficient T cell populations within limited periods of time. Lundin et al, (1989) also suggested that CD25-positive cells could be obtained after various stimulations but did not provide any explicit or useful result.

The present invention provides a method for obtaining antigen-specific T cells suitable both for epitope mapping, clinical applications and diagnostic purposes.

The present invention also provides methods of preparing compositions comprising antigen-specific T cells, for use in regulating an immune response in a subject, particularly in immunodeficient subjects, prior to, during or after organ transplantation, such as bone marrow transplantation.

The method according to the invention, for obtaining T-lymphocytes specific for known or unknown epitopes comprises the step consisting of:
(a) Stimulating T-lymphocytes with an antigen ;
(b) Isolating or purifying the stimulated T-lymphocytes using a marker of stimulation such as CD25 ;
(c) Amplifying said isolated or purified T-lymphocytes.

The T-lymphocytes stimulated with the antigen according to step (a) can be of any kind, and can be for example PBMC. They may be autologous or allogeneic (or even xenogeneic).

The specific T-lymphocytes which are amplified according to the method of the invention are useful for obtaining a specific cellular immune response.

An object of this invention is more particularly a method for obtaining T-lymphocytes specific for known or unknown epitopes, comprising the steps consisting of:
(i) Optionally depleting CD25-positive cells present in a sample of peripheral blood mononuclear cells (PBMC);
(ii) Incubating the PBMC or the CD25-negative PBMC with at least one antigen likely to contain at least one epitope ;
(iii) Isolating or purifying the CD25-positive cells which have appeared ;
(iv)Amplifying the isolated CD25-positive cells for example in the presence of IL-2.

The antigen may be of any kind. Said antigen may be selected from the group consisting of a peptide, a mixture of peptides, a protein, a naturally occurring target cell, a target cell transfected with a nucleotide vector coding for a peptide or a protein, a non-peptide antigen such as a hydrocarbon molecule, a cell infected by a virus or a bacterium, or a tumor cell, as well as fungi, pathological cells or any antigen against which a cellular immune response is desired.

Said antigen is preferably an integral protein. However one can also use a mixture of overlapping peptides, as the method of the invention allows to probe a relatively large protein area for the presence of T-cells epitopes.

Said antigen can be a molecule which derives from a virus or a tumor cell, which means that the antigen in its native form is expressed at the surface of a virus or a tumor cell and can be recognized by T-lymphocytes as an epitopes. The antigen is preferably all or part of a viral protein, such as a viral envelope protein or lytic protein. Specific examples include (i) the pp65 protein of CMV, particularly any fragment thereof of at least 5 consecutive amino acids comprising an epitope, more preferably at least 8 consecutive amino acids, such as SEQ ID NO:1-9 or 10 and (ii) the lytic protein BMLF1 of EBV or any fragment thereof, such as for instance any fragment of at least 5 consecutive amino acids, more preferably at least 8 consecutive amino acids, comprising SEQ ID NO:11 for instance.

Preferably the targeted epitope is involved in the activation of the T-lymphocytes, more particularly in the activation of CD4 T-lymphocytes that are called "helper" lymphocytes.

In a particular embodiment of the invention, said antigen is a cell infected by a virus (such as EBV), or a bacterium such as a mycobacterium. Said cells may be for example B-lymphocytes such as BLCL or a tumor cell or any other antigen presenting cell infected or transfected by a virus or vector encoding a viral antigen or protein (e.g., dendritic cells, macrophages, etc.).

According to the method of the invention, the depletion of CD25-positive cells (e.g., step (i)) can be effected by any standard technique well-known by one skilled in the art. For example, the PBMC can be isolated on a Ficoll gradient and the CD25-positive cells can then be sorted out by an immunomagnetic method (Thiel et al (1998)) by means of a column or by panning.

In a preferred embodiment, CD25-positive T cells are removed by contacting the cell population with a specific ligand of CD25, and isolation of ligand-bound cells. The ligand is preferably an anti-CD25 antibody or a fragment or derivative thereof. The antibody may be polyclonal or monoclonal, preferably monoclonal. Anti-CD25 antibodies are commercially available or can be produced by conventional immunization methods (Antibodies: A laboratory Manual, CSH Press, 1988 ; Kohler et al., Nature 256 (1975) 495, incorporated therein by reference). Specific examples of such antibodies include, for instance, monoclonal antibody produced by hybridoma 33B31. Isolation of antibody-bound cells (i.e., CD25-positive cells) can be accomplished by various techniques, including affinity columns, immuno-precipitation, use of a second antibody directed against the anti-CD25 antibody, said second antibody being coupled to a support (e.g., column, bead, etc.). In a preferred embodiment, the cells are first treated with an anti-CD25 antibody and then contacted with a capture antibody coupled to a magnetic bead. Depletion (or isolation) is then performed by applying a magnetic field, according to conventional methods. It is not necessary to remove 100% of CD25-positive cells to perform the method of the present invention. Preferably, at least 60% CD25-positive cells are removed, even further preferably, at least 80%.

Activation of T lymphocytes (e.g., step (ii)) of the method of the invention can be carried out by incubating the cells at high concentration (for example around 10 million cells per ml) with the antigen, whereby the cells are sensitized. These cells are then washed and grown at around 2,5 million cells per ml. This incubation can last for example between around 10 and 80 hours, preferably between around 18 hours and 72 hours.

The isolation step is aimed at purifying the newly appeared CD25-positive cells. It can be effected as described for the depletion step.

In the amplification step, the purified cells may preferably be cultivated in the presence of Interleukin 2. It is also possible to intensively restimulate them by further using feeder cells (such as autologous peripheral blood mononuclear cell (PBMC), more particularly the CD25-negative fraction or such as allogeneic PBMC and allogeneic B-lymphoblastoid cell line (BLCL)), and/or a polyclonal activator such as PHA (phytohemagglutinin).

This method provides an easy way for selecting T cells directed against a particular MHC + peptide complexe, given the fact that many antigens have already been identified.

This method also allows to systematically search for T-cells against still unknown epitopes.

For that purpose one can incubate the CD25-negative cells in step (ii) of the invention, with a mixture of overlapping peptides derived from a protein of interest.

The invention further provides a method for identifying an unknown epitope wherein the specific T-lymphocytes which were isolated and amplified as above described are further contacted with a fragment of said antigen, likely to contain said epitope, and the function (cytotoxic activity, cell proliferation, cytokine production) of said specific T-lymphocytes towards the fragment of the antigen is assessed, this functional assay being repeated with each overlapping fragment of said antigen, whereby the epitope fragment which triggers the functional activity of the specific T-lymphocyte towards the fragment of the antigen is identified.

In a preferred embodiment, the specific T-lymphocytes are contacted with target cells loaded with a fragment of the antigen likely to contain the epitope. This functional assay can be carried out by any standard method that one skilled in the art knows very well, such as a ⁵¹Cr release assay, ELISPOT, H³-thymidine incorporation, ...

In another embodiment, the specific T-lymphocytes are directly contacted with the fragments of the antigen. The lymphocytes present the antigen to each other in this particular case, and the cytokine production or the cell proliferation is then evaluated.

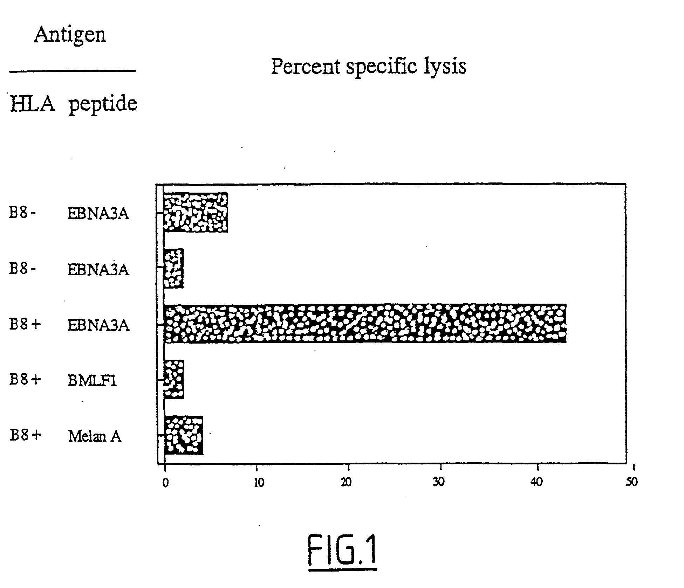

The authors of the invention have further shown that the method of the invention was particularly advantageous for rapid selection of T-cells stimulated with virus-infected cells. The proliferation of the specific T-cells selected according to the method of the invention is increased in comparison with standard procedures which further require several stimulations, as shown in figure 1.

In this regard, an object of this invention also resides in a peptide comprising a T cell epitope, wherein the peptide has a sequence of an epitope prepared or identified by the method as described above.

In a more specific embodiment, this invention also relates to the peptides selected from SEQ ID NO: 1-9, as well as variants or fragments thereof or larger peptides (i.e., of up to 100 amino acid residues) comprising all or part of the sequence of said peptides. Fragments include peptides comprising at least 5 amino acid residues, more preferably at least 8 consecutive amino acid residues, more preferably at least 9 consecutive amino acid residues. Examples of such fragments are underlined in Table 4.

A further object of this invention resides in a method of producing EBV-specific T-lymphocytes, the method comprising:
- treating a population of cells comprising T lymphocytes to remove CD25-positive cells,
- contacting the treated cell population with an EBV antigen to effect a stimulation of T lymphocytes, and
- isolating CD25-positive cells, wherein said cells comprise EBV-specific T-lymphocytes.

In a preferred embodiment, the EBV antigen is a cell infected by an EBV virus, preferably an antigen presenting cell infected by an EBV virus. The cell can be a population of (autologous) PBMCs infected with an EBV virus or an EBV-immortalized autologous B lymphoblastoid cell line.

In a further preferred embodiment, the CD25-positive cells are further cultivated to amplify or expand the population, for instance in the presence of interleukins (e.g., IL-2). Expansion can last for several days or weeks, as appropriate.

Another object of this invention is a method for producing antigen-specific T lymphocytes in vitro, the method comprising :
a) obtaining a population of cells comprising T lymphocytes,
b) treating said population of cells to remove CD25-positive cells,
c) contacting the treated cell population with an antigen to effect a stimulation of T lymphocytes, and
d) isolating CD25-positive cells from the cells of step c), wherein said cells contain T lymphocytes specific for said antigen.

This invention also encompasses methods of preparation of a composition to stimulate an immune response in a subject, said composition comprising antigen-specific T lymphocytes, the method comprising:
a) treating a population of cells comprising T lymphocytes to remove CD25-positive cells,
b) contacting the treated cell population with an antigen to effect a stimulation of T lymphocytes,
c) isolating CD25-positive cells from the cells of step c), and
d) conditioning the cells in a pharmaceutically acceptable diluent or carrier.

As indicated before, in the above method, CD25 may be replaced by another marker of T cell activation. In a particular embodiment, prior to step a), the population of cells is contacted with peripheral blood mononuclear cells from the subject to remove, after activation, the CD25-positive alloreactive T cells from said population. This prior step is advantageous since it avoids or reduces the risk of producing GVHD upon injection of the cells to the subject. In this regard, it is particularly preferred to treat the cells to remove or reduce alloreactive T cells. Indeed, during graft, the clinician will reduce T cells to minimize the risks of GVHD. This, however, increases the risk of infection and, when using the present therapeutic T cells, it is mostly advantageous to reduce potentially existing alloreactive T cells.

The present invention is also very advantageous since CD25-depletion appears to further increase the efficacy of the therapeutic T cells by removing suppressive T cells from the composition. Indeed, certain immuno-suppressive T cell clones have been shown to express CD25 marker. By depleting the same during cell processing, the invention thus increases the immunogenic power.

In a preferred embodiment, the antigen is a viral antigen, preferably selected from an EBV or a CMV antigen. Most preferably, the antigen is all or an immunogenic fragment of EBV-early lytic protein BMLF1 or of CMV envelope phosphoprotein pp65. The antigen is advantageously presented by an antigen-presenting cell, such as a B cell or PMBCs. In that case, the presenting cells may be prepared by infection of the cell with the virus, or by contacting the cell with a vector encoding all or a portion of a viral protein, or by loading the cell with one or several peptides from the virus.

An other object of this invention is a method of stimulating an antigen-specific immune response in an immunodeficient subject, the method comprising:
a) treating a population of cells comprising T lymphocytes to remove CD25-positive cells,
b) contacting the treated cell population with an antigen to effect a stimulation of T lymphocytes,
c) isolating CD25-positive cells from the cells of step c),
d) optionally, expanding the population of CD25-positive cells by in vitro culture,
e) conditioning the cells in a pharmaceutically acceptable diluent or carrier, and
f) injecting the population of CD25-positive cells to the subject.

The invention also resides in a method of preventing or reducing viral infection in a subject during or after bone marrow transplantation, the method comprising injecting to a subject at risk of developing viral infection a composition comprising T cells specific for a virus, said composition being obtained by:
a) treating a population of cells comprising T lymphocytes from a donor subject, to remove CD25-positive cells,
b) contacting the treated cell population with a viral antigen to effect a stimulation of T lymphocytes,
c) isolating CD25-positive cells from the cells of step c),
d) optionally, expanding the population of CD25-positive cells by in vitro culture, and
e) conditioning the cells in a pharmaceutically acceptable diluent or carrier.

Preferably, the injected cell population comprises at least 10² T cells, more preferably at least 10³ T cells, even more preferably at least 10⁴ T cells. Injection can be performed in one or repeated injections, depending on the clinical condition and subject. The injection can be performed using known devices (serynge, perfusion, etc.) and according to various routes (e.g., intravenous, intra-arterial, intra-dermic, sub-cutaneous, etc.).

The present invention is particularly suited for the preventive or curative treatment of EBV infection in patients undergoing immuno-suppression. In particular, patients receiving organ transplants, and particularly bone marrow transplantation, kidney transplantation or heart transplantation, are first subjected to immunosuppressing treatments and/or medullary aplasia. This is the case for instance with patients having blood cell tumors such as leukaemia for instance. These patients are treated to destroy their own blood or immune cells, prior to receiving bone marrow transplantation. However, under such induced immuno-suppression status, the patients have a risk of developing viral infections, particularly EBV, CMV and/or adenovirus infections or activations. In bone marrow transplantations (BMT), EBV infection can very rapidly lead to a lethal leukaemia in the patients. In addition, while CMV infections are usually treated by anti-viral compounds such as ganciclovir, these treatments are expensive and do not always suffice to protect the patients.

Following BMT, patients have a high risk of developing EBV infection or reactivation from their own infected cells or from the donor cells. The risk is about 5-20% for patients having the following criteria : important conditioning, major immune mismatch or severe T-cell depression. Because these patients have a high risk of developing GVHD, the clinician will reduce the amount of injected T cells during BMT and, most of the time, inject T-cell-depleted BM. The drawback associated with this approach is that the patient has a high risk of developing viral infection, because of the absence of protecting T cells. These patients are regularly monitored for their EBV status by PCR and, when about 3000 copies of the virus are detected per ml of blood, a treatment is required to protect the patient from lethal leukaemia, which can occur within 30 days. The present invention now proposes a novel strategy to treat or prevent such EBV reactivation in subjects undergoing bone marrow transplantation. The strategy is based on the injection of EBV-specific T cells prepared from the donor, according to the above method. Injection of such T cells, either prior to or upon reactivation of EBV in the subject, can significantly reduce or stop EBV progression by destroying infected cells in the subject.

In a particular embodiment, a composition of T cells as described above, prepared from the donor subject, is injected to the patient to prevent EBV activation. Preventive injection can be performed for patients at risk as defined above, particularly those having a major immune mismatch. Curative injection are performed when the EBV levels in the subject are above the threshold of about 3000 copies/ml.

To practice this method, about 10⁸-10⁹ PBMCs are collected from the donor subject prior to, during or after the bone marrow transplantation, for instance by lymphapheresis. A sample of these PBMCs (e.g., 10⁴ to 10⁷ PBMCs) are used to prepare an EBV-presenting cell, e.g., by infecting said sample with an EBV virus (attenuated or securized virus) or transfecting said cells with a recombinant vector encoding an EBV antigen or protein. These cells and the PBMCs are preserved, for instance under frozen state.

If, upon BMT, the patient exhibits viral activation above the threshold, the PBMCs can be treated as described above (e.g., depleted for CD25-positive cells) and contacted for about 24hrs to 72hrs with the irradiated EBV-presenting cells to effect stimulation and produce CD25-positive, EBV-specific T lymphocytes. As indicated above, in a preferred embodiment, the PBMCs may be contacted with PBMCs from the patient prior to CD25-depletion, in order to further remove or reduce alloreactivity. This treatment allows to eliminate endogenous CD25-positive cells from the donor, as well as CD25-positive cells which have been activated by the patients T cells. Following this method, between 10⁵ and 10⁶ CD25 positive T cells may be recovered. These cells (or a portion thereof) may be immediately injected to the patient, i.e., without expansion step. The present invention indeed proposes to inject activated T cells to the subject, immediately following stimulation, without in vitro expansion and re-stimulation steps. This is particularly advantageous since no in vitro culture and expansion is required. Alternatively, a portion of the cells may be injected and the rest may be subjected to in vitro expansion and injected at a later stage, if needed.

The efficacy of the method can be measured by the decrease in EBV copy number in the subjects' blood cells.

In a further alternative protocol, the EBV-presenting cells may be further contacted with a CMV antigen in order to produce T cell compositions specific for both EBV and CMV viruses.

The present invention may also be used to produce T cells specific for other viruses (e.g., hepatitis, such as hepatitis C, HIV, herpes virus, etc.).

The present invention can also be used to monitor the presence of antibodies or antigens in a subject, e.g., to assess the immune status of a subject or to follow the efficacy of a treatment.

The present invention also encompasses compositions comprising T lymphocytes prepared as described above, particularly pharmaceutical compositions. Acceptable diluents or carriers include buffer solution, isotonic solution, saline solutions, optionally comprising stabilizers, and the like. The compositions may be formulated in pouch, bags, flasks, etc.

The below figures and examples illustrate the invention without limiting its scope in any way.

### LEGEND TO THE FIGURES

**Table 4:** CMV-pp65 peptides recognized by the CD8+ or CD4+ T-lymphocytes clones derived from donors 8, 12, 15 and 20 after ALVAC-pp65 stimulation of CD25(-)-PBMC. Underlined sequences indicate the minimal peptide recognized by the clone, a and b indicate epitopes that have also been detected by others.

**Figure 1** represents the cytotoxic activity of T-cells selected against the EBNA3A peptide FLRGRAYGL. Results are expressed as percentage of cytotoxic activity against HLA-B8+ or HLA-B8- target BLCL loaded with 10 µM of the indicated peptide minus the background cytotoxic activity obtained against unloaded target cells. In that case the control peptides were HLA-A2 binding-peptides.

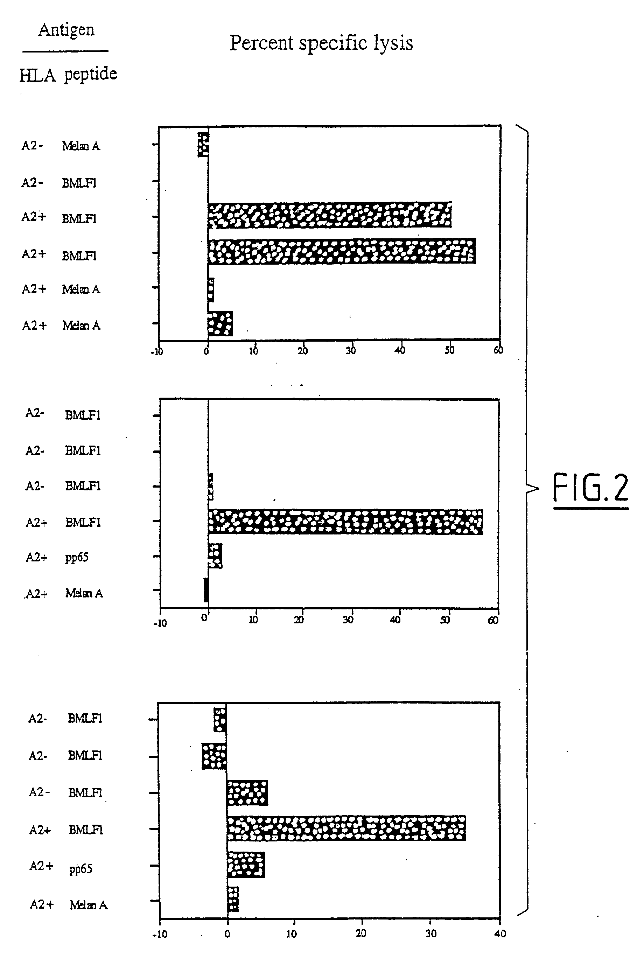

**Figure 2** shows the cytotoxic activity of T-cells selected against the BMLF1 peptide GLCTLVAML. Results are expressed as percentage of cytotoxic activity against HLA-A2+ or HLA-A2- target BLCL loaded with 10 µM of the indicated peptide minus the background cytotoxic activity obtained against unloaded target cells. Experiments a, b, and c were performed with PBMC from 3 different donors. In that case all peptides bind to HLA-A2.

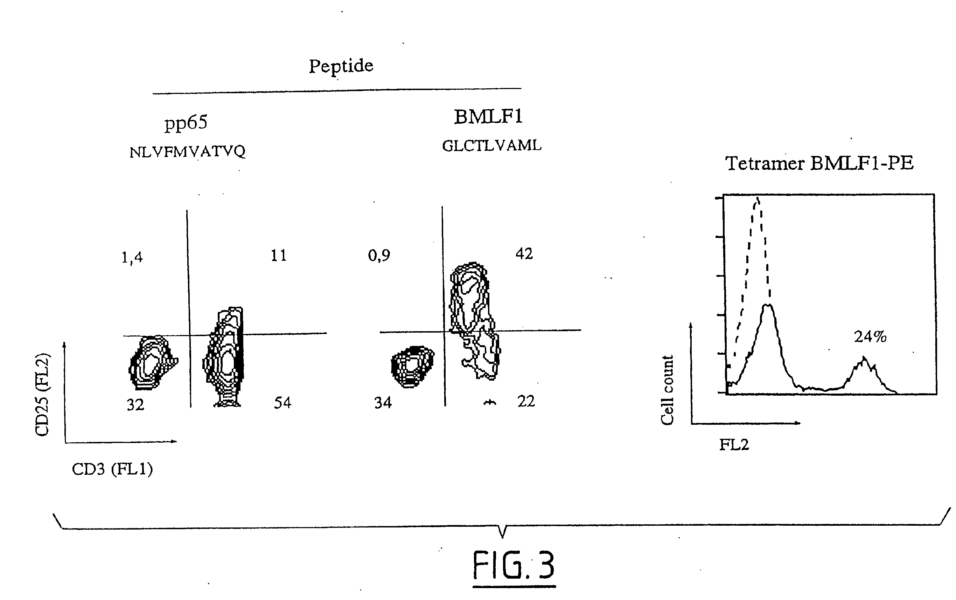

**Figure 3** is a purity estimation of a BMLF1 selected T-cell population. **a)** CD25 expression after stimulation with an HLA-A*0201 BLCL loaded with the BMLF1 or a control (pp65) peptide. The lower left spot corresponds to contaminating NK cells **b**) fluorescence analysis after staining of the BMLF1-selected PBMC with a phycoerythrin (PE) conjugated BMLF1-A2 tetrameric molecule. The negative control was performed with a PE-conjugated pp65-A2 tetrameric molecule

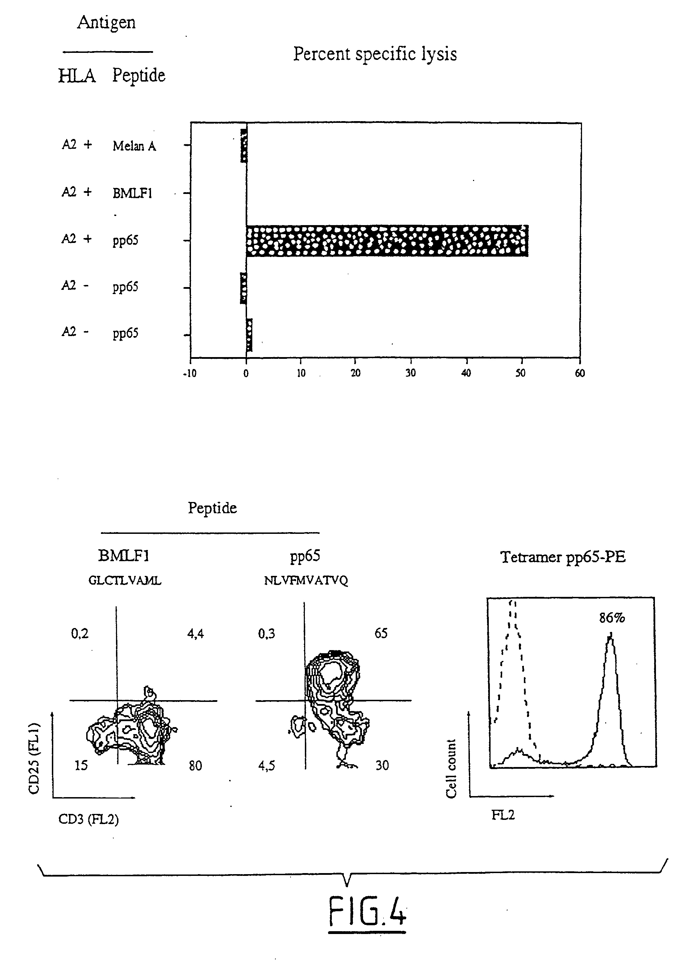

**Figure 4** shows the characterization of a pp65 selected PBMC population. Same as Figure 2 and Figure 3.

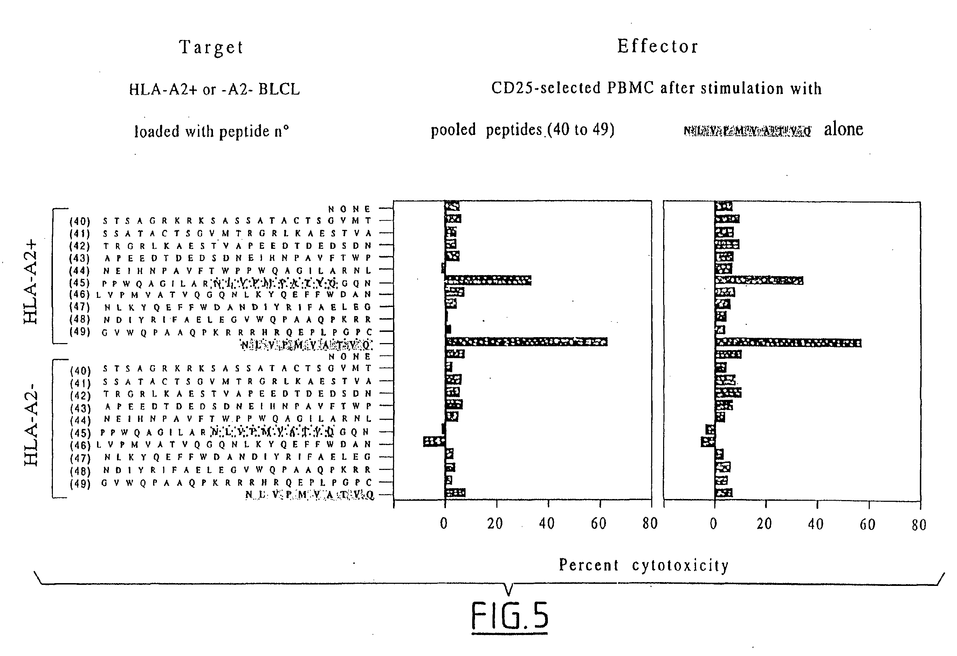

**Figure 5** shows the validation of the method for identifying new epitopes, by means of a pool of overlapping peptides. CD25-depleted PBMC from an healthy CMV seropositive donor were stimulated with a mixture of 10 overlapping peptides spanning 1/5 of the pp65 protein NH2-terminal region. Note that peptide n°45 includes the known immunodominant decamer pp6-IT. In line with our previous results, stimulation with pp65 495-504 alone was used as a positive control. Final peptide concentration was 10 µM for pp65 495-504 and 5 µM for the peptide pool. After 18h incubation at 37°, the CD25 positive cells were separated as described in the method section. After *in vitro* amplification CD25 selected PBMC were tested against HLA-A2+ and HLA-A2-BLCL loaded with the indicated peptide. Results are expressed as percentage of specific lysis, i.e. that obtained against peptide loaded BLCL minus that obtained against unloaded BLCL.

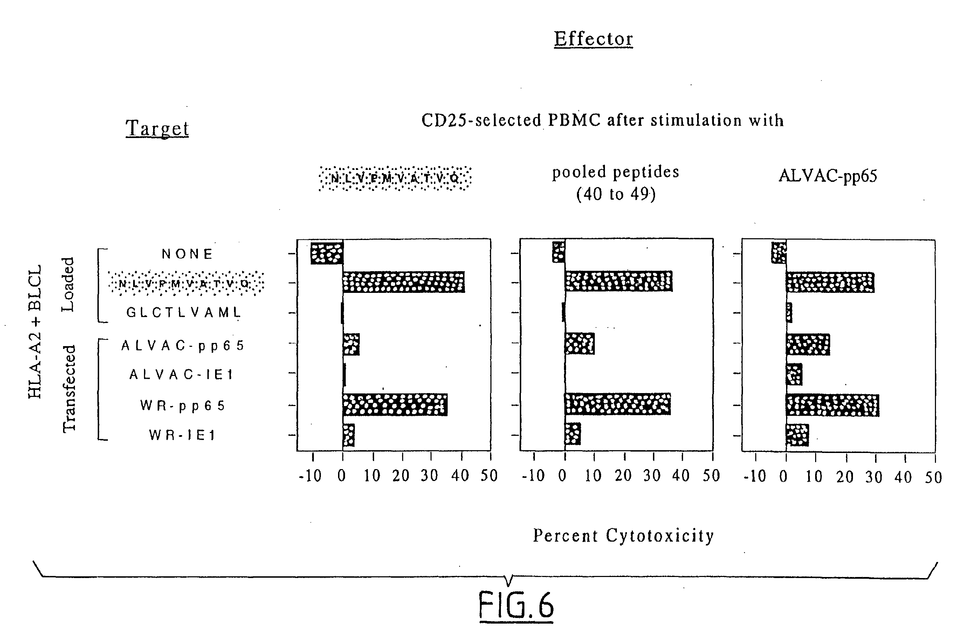

**Figure 6** shows the validation of the method for identifying epitopes by means of a viral vector encoding a protein of interest. CD25-depleted PBMC were infected for 60' at a multiplicity of infection of 10:1 and coculture with 80X106 PBMC for 18h before CD25 selection. This assay was performed in parallel with the other procedures described previously, i.e. a stimulation using the pool of peptides (n°40 to 49) or the peptide pp65-IT alone. After amplification, CD25 selected cells were tested against an HLA-A2+ BLCL either loaded with pp65 495-504 or a control peptide (GLCTLVAML derived from the EBV protein BMLF1) or infected with ALVAC-pp65, ALVAC IE1, or with the recombinant vaccinia viruses WR-pp65 or WR-IE1. Results are expressed as the percentage of specific lysis at an E:T ratio of -10/1.

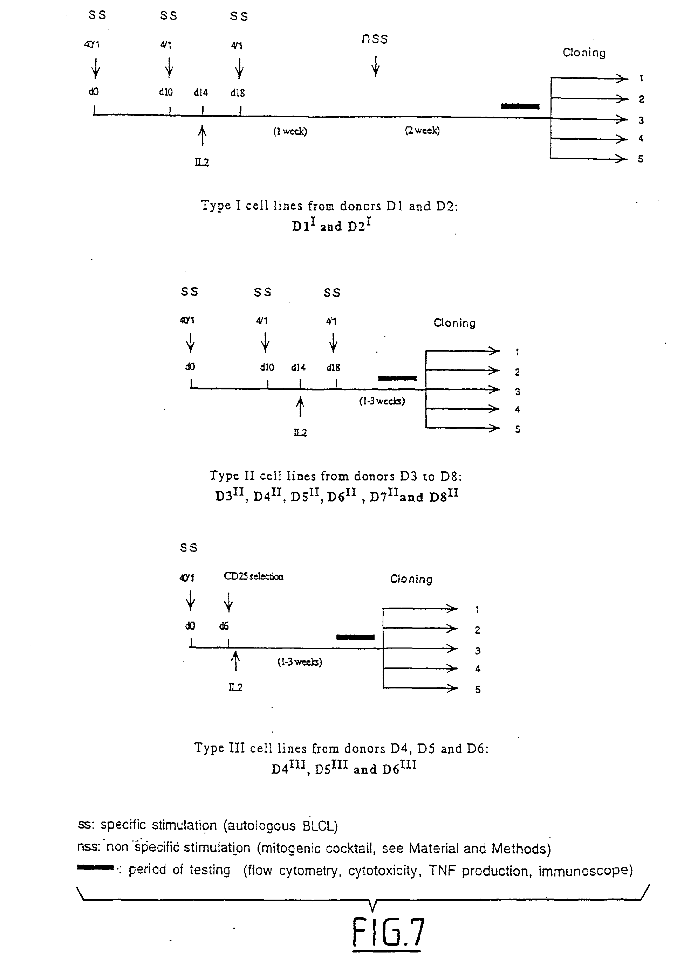

**Figure 7** represents the T-cell line selection protocol followed in Examples 4 to 6.

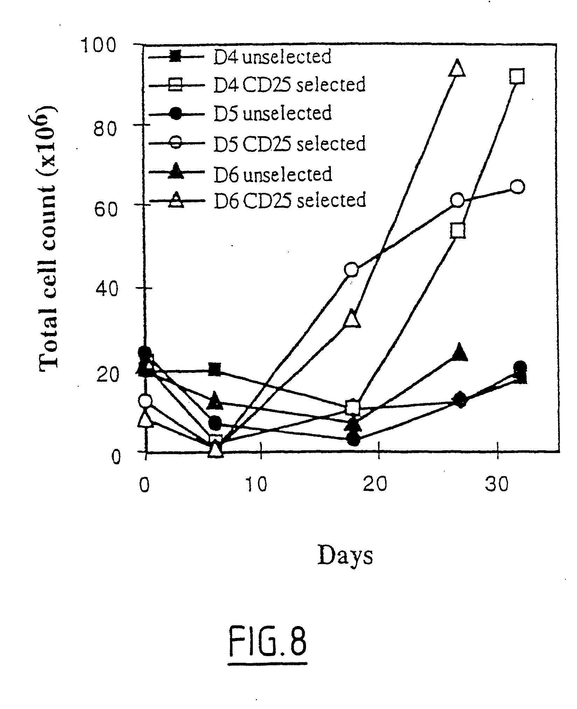

**Figure 8** is a comparison of growth kinetics for T-cells selected with the CD25-method of the invention vs unselected T-cells (D4^{II} vs D4^{III}, D5^{II} vs D5^{III} and D6^{II} vs D6^{III}).

### EXAMPLES

### Examples 1-3

Using purification of various EBV or CMV antigen-specific T-lymphocytes as a model, the authors of the invention tested also different possibilities of PBMC stimulation before the CD25 selection i) with a single small peptide (corresponding to an epitope already identified) in order to prove the concept ii) with a pool of 10 large overlapping peptides (23 aa long with a step of 12) in order to select T-cells specific for unique or several contiguous known epitopes or to search for new epitopes on a chosen protein area and iii) by infecting PBMC with a replicative defective Canarypox (ALVAC) vectors encoding the entire pp65 protein to select the T-cell repertoire specific for this protein.

### Material and methods:

**Donors :** Blood packs were obtained from 7 healthy adult donors after informed consent. Three were HLA-A*0201, EBV+ (Donors 1, 2 and 3), one was HLA-B8, EBV+ (Donor 4) and three are HLA-A*0201, CMV+ (Donors 6, 8 and 12). Peripheral blood mononuclear cells (PBMC) were separated using Ficoll density centrifugation (lymphocyte separation medium, Eurobio, France).

**Table 1.**

| HLA Typing of the Donors and Panel B Lymphoblastoid Cell Lines (BLCL) | | | | | | |
|---|---|---|---|---|---|---|
| CODE | BLCL | HLA-A | HLA-B | HLA-DR | HLA-DQ | HLA-DP |
| A | BM9 | 2.2 | | | | |
| B | BOIS | 24 | 40/35 | | | |
| C | IBW9 | 33.1 | w65 | 7 | 0501 | 0101 |
| D | SPOO10 | 2.2 | 44.2 | 1101 | 0502 | 02012 |
| E | VAVY | 1 | 8 | 0301 | 0201 | 0101 |
| F | SYL | 3 | 8/18 | 1403/3 | 2/503 | 401/201 |
| Do1 | Donor 1 | 201 | | 16/03 | 05/02 | 0401/1101 |
| Do2 | Donor 2 | 201 | | | | |
| Do3 | Donor 3 | 201 | | | | |
| Do4 | Donor 4 | | | | | |
| Do6 | Donor 6 | | | | | |
| Do8 | Donor 8 | 1/2 | 51 | 1302/08 | 06/04 | 0401/1401 |
| Do12 | Donor 12 | 02/33 | 08/14 | 01/15 | 0501/0602 | 0401/1101 |

**Peptides :** The following peptides were obtained > 70% pure by HPLC from Genosys, Cambridge, UK: The HLA-A2 binding peptide AAGIGILTV (referred to as A9V) derived from the melanoma associated MelanA/MART-1 protein (Fleschhauer et al, (1996)), the HLA-A2 binding peptide NLVPMVATV (referred to as N9V ) derived from the pp65₄₉₅₋₅₀₄ CMV matrix phosphoprotein (Burrows et al, (1992)), the HLA-A2 binding peptide GLCTLVAML (referred to as G9L), derived from the EBV early lytic protein BMLF1 and the HLA-B8 binding peptide FLRGRAYGL (referred to as F9L) derived from the EBV latent protein EBNA3A (Altman et al, (1996)): In addition a panel of fifty 23-amino-acid (23mer) peptides (numbered 1 to 50) overlapping by 12 amino-acids and spanning the entire CMV pp65 sequence (aa 1 to 562) was obtained from Chiron Mimotopes, Suresnes, France. Note that the immunodominant HLA-A2 decamer NLVPMVATV was included in the 23aa long peptide # 45. Peptide stock solutions (20 mg/ml in DMSO) were diluted first to 2 mg/ml in acetic acid (0,1%) and second to the final concentration in RPMI1640 culture medium (Sigma-Aldrich, St Quentin Fallavier, France). See Table I for peptides references and nomenclature.

**CD25+ depletion of fresch PBMC:** The small fraction of CD25 positive cells already present among unstimulated PBMC was depleted as follow : fresch PBMC were incubated at a concentration of 2x10⁸/ml during 20 mn at 4°C in phosphate-buffered saline (PBS) containing 5% pooled human serum (HS), 2 mM EDTA and 20 µg/ml of the anti-CD25 moAb 33.B.3.1. Cells were then washed twice with 30 ml of cold PBS/HS/EDTA (centrifugations were performed at 4°C at 300xg) and the pellet resuspended in cold PBS/HS/EDTA (80 µl for 1x10⁷ cells). Goat anti-rat MicroBeads (Miltenyi Biotec, Begisch Gladbach, Germany) were added (20 µl for 1x10⁷ cells), and the cell suspension mixed gently and incubated for 15 mn at 4°C. The cells where then washed in 25 ml of cold PBS/HS/EDTA (centrifugations were performed at 4°C at 300xg without brake) and resuspended in the same buffer (500 µl for 1x10⁸ or less cells). Depletion of CD25 positive cells was performed using the VarioMACS with an AS column (Miltenyi Biotec, Begisch Gladbach, Germany) according to the supplier's instructions.

**Stimulation of CD25 depleted-PBMC with peptides:** The CD25-depleted PBMC fractions were loaded for 2 h at 1x10⁷/ml with 1µM of BMLF1 (Do1, Do2 and Do3), 1,25 µM of EBNA3A (Do4), 10 µM pp65₄₉₅₋₅₀₄ (Do6, Do8 and Do12) or 5 µM of 40 to 49 mixture peptides (Do8 and Do12) in RPMI1640 alone in 15 ml polypropylene tube (Sarsted Inc, Newton, NC). Then, cells washed twice and cultured in flasks TC 80 cm² (Nunc, Copenhagen, Denmark) at 2,5x10⁶/ml in RPMI1640 + 8% HS + 1% L-glutamine + 50 µg/ml gentamicin without cytokine for 24-96h.

**Stimulation of CD25 depleted PBMC with ALVAC (canarypoxvirus):** 1/4 of the CD25- PBMC fractions (Do8 and Do12) were infected at 1x10⁷/ml with recombinant ALVAC-pp65 expressing the pp65 matrix protein (Virogenetics, Troy, NY), for 60 mn at 37°C in RPMI1640 alone in 15 ml polypropylene tube (Sarsted Inc, Newton, NC) at a multiplicity of infection (MOI) of 5:1. Then, cells washed once and co-cultured with the 3/4 remaining CD25-PBMC in flasks TC 80 cm² (Nunc, Copenhagen, Denmark) at 2,5x10⁶/ml in the same medium described above (RPMI1640 + 8% HS + 1% L-glutamine + 50 µg/ml gentamicin without cytokine) for 24-72h.

**Selection of antigen specific T cells**: After 24-96h of stimulation with peptides or recombinant ALVAC-pp65, cells were incubated with the anti-CD25 in the same conditions as for the CD25 depletion (ie 2x10⁸ cells/ml with 20 µg/ml 33.B.3.1 rAb, 20 mn at 4°C), washed twice and incubated with Goat anti-rat MicroBeads (ie 80 µl of cold PBS/HS/EDTA and 20 µl of Miltenyi's Goat anti-rat MicroBeads for 1x10⁷ cells, 15 mn at 4°C), washed -once and resuspended in 500 µl of cold PBS/HS/EDTA for 1x10⁸ cells and 500 µl for less than 1x10⁸ cells. A CD25 positive selection was performed using the VarioMACS on a MS+/RS column (Miltényi Biotec, Begisch Gladbach, Germany) according to the supplier's instructions. The CD25 positive fraction was then stimulated with pooled allogeneic feeder cells (5x10⁶ irradied (35 Gys) PBMC and 5x10⁵ irradied (35 Gys) B lymphoblastoid cell lines (BLCL), in the presence of 1 µg/ml of leukoagglutinin-A (Sigma, St Louis, Missouri, USA) and 150 BRMP U/ml of rlL2 (Proleukin, Adesleukine, Chiron BV, Amsterdam, Pays-Bas). Before specificity assays, cells lines were cultured without stimulation in rIL2 alone (150 BRMP U/ml) for at least 3-6 weeks.

**Isolation of CD4+ cells:** 5x10⁶ T cell lines were incubated with anti-CD4 mAb diluted at 1/40 (BioAtlantic, Nantes, France) 30 mn at 4°C, washed twice, and incubated at a 4:1 bead-to-cell ratio with Dynabeads M-450 Sheep anti-Mouse IgG (Dynal, Oslo, Norway) according to manufacturer's instructions. The CD4+ fraction was then stimulated with pooled allogeneic feeder cells, in presence of leukoagglutinin-A and rlL2 as described above. Before specificity assays, the cells lines were cultured in rlL2 alone (150 BRMP U/ml) for at least 3-6 weeks.

**Cloning** : To generate a panel of clones from T cell lines, one responder T cell was seeded in every three culture wells in 96-microwell round bottom culture plate (Nunclon, Copenhagen, Denmark) together with pooled allogeneic feeder cells (5x10⁴ PBL and 5x10³ BLCL, 35 Grays irradiated) in the presence of leukoagglutinin-A (1µg/ml), and rlL-2 (150 BRMP U/ml). Before specificity assays, the clones were cultured without stimulation in IL2 alone for at least 2 weeks.

**Generation of EBV-transformed B cell lines:** Autologous BLCL were generated for each donor in the following conditions : 10x10⁶ PBMC were cultured at a density of 2x10⁶ cells per well in a flat-bottomed 24-well plate in 100 µl of RPMI 1640 containing 10 % fetal calf serum (FCS), 1 % L-glutamine (2 mM) and 50 µg/ml gentamycin in the presence of 0.1 µg per ml of cyclosporine A and 500 µl per well supernatant derived from cultures of B95-8, a marmoset B cell line transformed by human type 1 EBV. After 24h, 2 ml of RPMI 1640 containing 10 % FCS, 1 % L-glutamine and 50 µg/ml gentamicin were added per well.

**Cytotoxicity assay**: T cell are taken more than 3 weeks after the last stimulation. The target cells were labeled with 100 µCi Na₂⁵¹CrO₄ for 1h at 37°C, washed three times. Target cells (Autologous or allogeneic BLCL and PHA-blasts) were either infected with recombinant vaccinia viruses (Virogenetics, Troy, NY), expressing the pp65 protein (WR-pp65) or IE1 protein, an immediate-early protein of the CMV (WR-IE1), at 1x10⁷/ml in RPMI1640 alone, MOI=10/1 for 60 mn at 37°C in 15 ml polypropylene tube (Sarsted Inc, Newton, NC) and then diluted to 8x10⁵ cells/ml in RPMI1640 + 10% SVF for overnight incubation. The next day infected cells were centrifuged and labeled with Na₂⁵¹CrO₄. Or target cells labelling with Na₂⁵¹CrO₄ were loaded with peptide (BMLF1 (10□µM), EBNA3A (10□µM), Melana/MART-1 (50 µM), pp65495-504 (10 µM), 40 to 49 mixture peptides (5 µM), peptides of the bank (10 µM), T12Y (10 µM) or L12Q (10 µM)) and washed twice. Target cells were plated at the indicated effector-to-target ratios in a 96-well round-bottom plate. After 4h of incubation at 37°C, 25 µl of supernatant from each well was removed and counted in a beta scintillation counter. Each test was performed in triplicate. Results are expressed as percentage of lysis, according to the following formula : (experimental release - spontaneous release) / (maximal release - spontaneous release) x 100, where experimental release represents mean counts per minute released from the target cells in the presence of effector cells, spontaneous release that from target incubated without effectors, and maximum release that from target incubated with 1% triton x100.

**Proliferation assays** : Resting T cells taken more than 3 weeks after the last stimulation were cocultured in 96-microwell flat-bottomed culture plates at a 1:1 responder-to-stimulator ratio for 72 h with the irradiated (35 Gys) autologous or allogeneic target BLCL described above. Six hours before harvesting, 1 µCi of (³H)thymidine was added to each well, and (³H)thymidine uptake was then measured in a liquid beta scintillation counter. Results are expressed as the mean of triplicate cultures.

**Immunoscope analysis** (Pannetier et al (1993) a) and b) : RNA was extracted as previously described. This technique involves a combination of PCR and run-off reactions using pairs of V / C primers followed by size determination of the elongation products. Fluorescent DNA products were migrated on sequencing gels in an automated DNA sequencer (Applied Biosystem. Foster City, CA) and raw data were analyzed by the immunoscope software package.

### Results:

### Example 1 :

### Purification of specific T-lymphocytes after stimulation of CD25-depleted PBMC with a single peptide:

Responder preparation (CD25-depleted), peptide stimulation and CD25-positive cell recovery: Because the frequency of T-cells specific for a single MHC+peptide complex is expected to be low, the few PBMC already expressing the CD25 antigen (0.2 to 2%) had first to be eliminated. After seven CD25 depletion where 3.3 to 8 x 10⁸ PBMC were CD25 depleted, the recovery varied from 56 to 87% (77+/- 12% in mean).

In preliminary experiments the authors of the invention tested the effect of different peptide stimulation conditions on total CD25+ recovery. The CD25-depleted peptide loaded fraction (2.5 or 10 µM) was either pelleted or adjusted to 10⁷ cell/ml for 2h before incubation (18 or 72h at 2.5 x 10⁶/ml). Cells were then either incubated in RPMI supplemented with 5% human serum (HS) or in X-vivo 15 serum free culture medium. As a negative control CD25-unloaded cells were in some cases also processed.

Through 6 experiments performed with the peptide BMLF1 the CD25+ frequencies varied from 1/600 to 1/286 (1/540 in mean), while for the 6 negative controls, CD25+ recovery varied from 1/800 to 1/300 (1/466 in mean).

Selection of T-cells specific for the EBV latent protein EBNA3A derived nonamer F9L presented by the HLA-B8 class-I molecule: In the experiment shown in Fig. 2, 388.000 (i.e. 1/257) CD25+ cells were recovered after incubation of 10⁸ CD25-depleted HLA-B8 PBMC with the peptide F9L (2.5 µM). After amplification using a procedure which preserves the initial diversity of the T-cell population amplified (Gaschet et al, (1996)) the CD25+ fraction was tested for cytotoxic activity against HLA-B8- or HLA-B8+ target BLCL in the presence of the stimulating F9L or of the G9L or A9V irrelevant peptides. The CD25-selected fraction killed the HLA-B8+ but not the HLA-B8- target BLCL loaded with F9L and did not kill the HLA-B8+ target BLCL loaded with A9V or G9L. Consequently, this T-cell population contain CTL specific for the HLA-B8/F9L antigenic complex. Note that all BLCL used in this study were obtained by transformation with the EBV strain derived from the marmoset B95.8 cell line, which encodes an equivalent EBNA3A epitope (F9I instead of F9L) not recognized when endogenously presented (Altman et al, (1996)). This is the reason why, HLA-B8+ BLCL are not recognized when they are not loaded with the wild type peptide.

Selection of T-cells specific for the EBV early lytic cycle protein BMLF1 derived nonamer G9L presented by the HLA-A*0201 class-I molecule: Stimulation conditions were the same as those described above for EBNA3A-B8 but for the peptide concentration (10 µM in the present case). For the 3 experiments presented in Fig. 2, frequencies of CD25+ cells recovered after 72h were respectively 1/286, 1/454 and 1/600. The CD25-selected population showed specific recognition of the HLA-A*0201/G9L antigenic complex only since neither the HLA-A*0201+ target BLCL loaded with the Melan-A or pp65 peptides (A9V and N9V) nor the HLA-A*0201 negative target BLCL loaded with the G9L peptide were recognized. In that case again it was not expected that unloaded BLCL be recognized by G9L specific CTL since among LCL cells only a small minority express proteins of the lytic cycle.

Selection of T-cells specific for the CMV envelop protein pp65 derived nonamerN9V presented by the HLA-A*0201 class I molecule: For this experiment, after incubation with N9V (10 µM), 50 x 10⁶ stimulated CD25-PBMC were loaded on the positive separation column and 366.000 CD25+ cells were recovered (i.e. 1/136). After amplification the CD25 selected fraction was tested for specificity and purity as previously described. Results are shown in Fig. 4. As one can see, this CD25 selected population was specific for the HLA-A*0201/ N9V antigenic complex only (Fig. 4a).

### Example 2:

### Selection of pp65-IT specific T-lymphocytes after stimulation of PBMC with a mixture of peptides.

The above data proved the procedure efficient and consequently validate the concept of PBMC stimulation with a single peptide followed by a CD25-selection step for the recovery of T-cells with known specificity. Next, we reasoned that if the epitope could still be recognized when presented among many others, then such procedure may potentially be used for the random search of new specificities. To test this hypothesis selection of HLAA*0201/ N9V specific T-lymphocytes was used as a model: PBMC from a CMV seropositive individual were stimulated in the condition described above but with a mixture of ten 23 mer (5 µM) spanning 1/5 of the pp65 protein instead of the single N9V sequence. Two sequences corresponding to epitopes frequently recognized by PBMC of CMV seropositive individuals were present among this set of peptides: the HLA-A*0201 restricted N9V sequence within the 23 mer n°45 and the HLA-B8 restricted Q15A sequence within the 23 mer n°47. As a control, PBMC were also stimulated with the N9V nonamer alone. After amplification, CD25-selected PBMC were tested against HLA-A2+ and HLA-A2- BLCL loaded or not with each of the 23 mer present within the pool used for stimulation, or with N9V alone. No response was observed against loaded or unloaded HLA-A2 negative BLCL (Fig. 5). No response against any target was detected with the CD25-selected fraction not stimulated with the peptide pool. In contrast, PBMC stimulated with the mixture of 23 mer peptides showed two readily detectable cytotoxic responses: one against HLA-A2+ BLCL loaded with the 23 mer n°45 (which contain the N9V sequence) and the other against HLA-A2+ BLCL loaded with the decamer N9V alone. Consequently, these data demonstrated that the decameric epitope N9V, even when it is included within one of 10 overlapping 23 mer, could still be spotted by HLA-A*0201/N9V -specific T lymphocytes so that they express the IL2 receptor alpha-chain (CD25). Such procedure seems particularly attractive for the screening of a limited set of peptides covering a limited protein area.

### Example 3:

### Selection of N9V specific T-lymphocytes after stimulation of PBMC with a Canarypox viral vector ALVAC-pp65 encoding the entire pp65 sequence.

Finally, using the CD25-selection protocol of the invention and with in mind the objective to apply such strategy in a clinical setting theauthors of the invention used a Canarypox viral vector to induce expression of the entire pp65 protein among fresh PBMC. Naturally attenuated canarypox (ALVAC) constructs have been shown to be efficient tool in the induction of protective immunity *in vivo* (Cadoz et al, (1992) ; Abimiku et al, (1995)) and also in the *ex vivo* activation of cytotoxic T lymphocytes (Ferrari et al, (1997)). These vectors, which retain the pancytotropism of most poxviruses, are unable to productively replicate in non avian species, and thus eliminate the safety concerns that exists for vaccinia vectors. Twenty millions PBMC were infected for 60' at a multiplicity of infection of 10:1 and coculture with 80X10⁶ uninfected autologous PBMC for 18h before CD25 selection. This assay was performed in parallel with the other procedures described above, i.e. a stimulation using the pool of peptides (n°40 to 49) or the peptide N9V alone. After amplification, CD25 selected cells were tested against an HLA-A2 BLCL either loaded with a peptide ( N9V or a control peptide) or infected with a canarypox vector (ALVAC-pp65 or ALVAC IE1), or infected with a recombinant vaccinia virus (WR-pp65 or WR-IE1). Results are reported in Fig. 6 as the percentage of specific lysis observed at an E:T ratio of 10/1. For the 3 cases, CD25-selected PBMC recognized the HLA-A2 BLCL when loaded with the N9V but not with the G9L control peptide. They also recognized the same target BLCL when it was infected with the recombinant vaccinia virus WR-pp65 but not with the recombinant virus expressing the immediate early protein IE1. As noted by others, Canarypox vectors are poor targeting vectors to infect BLCL (Ferrari et al, (1997)): accordingly, although HLA-A2 BLCL infected with the ALVAC-pp65 showed some recognition compared to ALVAC-IE1 infected BLCL, the level of cytotoxicity observed was well below that obtained against recombinant vaccinia virus infected BLCL. Remarkably, the 3 CD25 selected cultures had a comparable level of cytotoxic activity against the target cells either loaded with a single peptide or infected with a vector encoding the entire protein. Although further analysis at the clonal level will be necessary to document the different specificities present among T-cell population selected with the pool of peptide and the ALVAC vector, this result already stressed the dominance of the response directed at the N9V CMV epitope.

At whole, specific T-lymphocytes were isolated after stimulation with pp65 495-504, peptide pool or ALVAC-pp65 from 3/5 (6, 7, 8, 11, 12), 2/3 (7, 8, 12), and 2/4 (7, 8, 11, 12) respectively.

### Examples 4-6 :

The authors of the present invention further used the method as above described to select T-cells which are EBV-specific.

For comparison purposes, six lines were selected using the standard protocol already used by others for clinical application (I- and II-type cell lines), and two lines were selected using the new method of the invention which required a single stimulation against autologous BLCL followed by a separation at day 6 of the CD25 positive activated T-cells (III-type cell lines).

### Materials and Methods

**Donors:** Fifteen milliliters of heparinized blood were collected twice from 8 healthy EBV-seropositive adults (D1 to D8). PBMC were separated using Ficoll density centrifugation (Lymphocyte Separation Medium, Eurobio, France). For EBV-transformed BLCL establishment, PBMC were cocultured with EBV-containing supernatant from the B95.8 EBV-producing cell line: 10 x 10⁶ PBMC were cultured at a density of 10⁶ cells/ml in a 24-well plate in RPMI 1640 + 10% FCS + 2 mM glutamine + gentamicin (50 µg/ml), initially supplemented with 0.1 µg/ml cyclosporin A and 500 µl/well of B95.8 culture supernatant.

**Generation and expansion of EBV-specific cytotoxic T-cell lines (Fig. 7)**: For type I cell lines (D1^{I} and D2 ^{I}), donor PBMC were plated in 24-well culture plates in RPMI 1640 supplemented with 10% FCS, 1% L-glutamine and 50 µg/ml gentamicin at 2 x 10⁶ cells per well and stimulated with 5 x 10⁴ 35 Gy irradiated autologous BLCL (PBMC/BLCL ratio of 40:1). After 10 days, T cells were harvested on Ficoll gradient and restimulated at a T / B ratio of 4:1 (5 x 10⁵ T and 1.25 x 10⁵ BLCL per well). IL-2 (150 BRMP U/ml) was added 4 days after the second stimulation, and a third stimulation in the presence of IL-2 was performed 8 days after the second one with the same T/B ratio (4:1). Ten days after this last specific stimulation, cultures were fed with a mitogenic cocktail composed of irradiated pooled allogeneic feeder cells (5 x 10⁴ PBMC and 5 x 10⁴ BLCL) in the presence of 1µg/ml leukoagglutinin A (Pharmacia, Uppsala, Sweden) and rlL-2 (150 BRMP U/ml). This procedure is sometimes required to reach the number of cells necessary for injection (Smith et al, 1995). Type II cell lines (D3^{II}, D4^{II}, D5^{II}, D6^{II}, D7^{II} and D8^{II}) were studied after the 3 specific stimulation steps, using the autologous BLCL. For type III cell lines (D4^{III}, D5^{III} and D6^{III}) after a 6-day coculture period of PBMC with BLCL (40:1 ratio), cells recognized by 33B3.1 mAb (an anti-CD25 mAb, were purified as follows: (i) 8 to 22 x 10⁶ cells were first stained with the 33B3.1 mAb (20 µg/ml) in 500 µl PBS (0.1% BSA) for 30 min at 4°C; (ii) cells were then washed twice in 10 ml sterile PBS-BSA; (iii) 1 x 10⁵ magnetic beads (Dynabeads M450, Dynal, Oslo, Norway) prepared according to the supplier's instructions were then added to the cell suspension and rotated for 4 h at 4°C; (iv) bead-coated and uncoated cells were then separated using a magnet (six washes were performed to ensure elimination of all uncoated cells). CD25-selected T-cells were then further cultured in the presence of IL-2 only.

**Cytotoxic assay:** Cytotoxic activity was tested using a standard 51Cr release assay. Briefly, target cells were labeled with 100 µCi Na₂⁵¹CrO₄ for 1 h at 37°C, washed four times and then plated at effector-to-target ratios of 3:1, 10:1 and 30:1 in a 96-well round-bottom plate. After 4 h of incubation at 37°C, 25 µl of supernatant from each well were removed and counted in a gamma scintillation counter. Each test was performed in triplicate. Results are expressed as percentage of lysis, according to the following formula: (experimental release - spontaneous release) / (maximal release - spontaneous release) x 100, where experimental release represents mean counts per minute released from target cells in the presence of effector cells, spontaneous release that from targets incubated without effectors, and maximum release that from targets incubated with 1% Cetavlon.

**Expression Vectors.** Expression vectors encoding 6 lytic EBV proteins (BZLF1, BMLF1, BRLF1, BCRF1, BMRF1, BHRF1), all the latent EBV proteins (EBNA-1, -2, -3a, -3b, -3c, -LP, LMP1 an LMP2), and various HLA class I alleles (HLA-A*0101, -A*0201, -A*0301, -A*2402, -B*0702, -B*0801, - B14, -B18, -B*2705, -B*3501, -B*4402, -B*4403, -Cw*0102, -Cw4, -Cw6, - Cw7, -Cw8, -Cw14,-Cw15 and -Cw16) were previously described (Scotet et al, 1996 ; Scotet et al, 1999).

**COS transfections and T-cell stimulation assay:** Transfection into COS cells-was performed by the DEAE-dextran chloroquine method, as described (Scotet et al, 1996 ; Brichard et al, 1993). Briefly, 1.5 x 10⁴ COS cells were cotransfected with 100 ng of an expression vector coding for an EBV protein and 100 ng of an expression vector coding for one of the HLA class I molecules. Transfected COS cells were tested 48 h after transfection in a CTL stimulation assay, using either clones or polyclonal cell lines. For clonal analysis, 5 x 10³ cells from the T-cell clone were added to transfected COS cells. Culture supernatants were harvested 6 h later and tested for TNFα content by measuring culture supernatant cytotoxicity to Wehi 164 clone 13 in a colorimetric assay. For polyclonal analysis, TNFα secretion in culture supernatant was estimated as for T cell clones, after 6-h incubation of varying numbers of polyclonal cell lines (10³, 10⁴ and 10⁵) together with transfected COS cells (Scotet et al, 1999).

### Example 4:

**Selection of EBV-specific T-cell lines:** Type I, II and III T-cell lines were obtained from 8 different donors (D1-8) according to the procedures described in Fig. 7 and in the method section. I and II-type cell lines were obtained using the standard selection procedure described by Heslop et al (1994) and which rely on sequencial stimulation of donor PBMC against autologous BLCL. In contrast, for type-C cell lines, only the first stimulation against auto-BLCL was performed (at a 40 to 1 responder to stimulator ratio) and the CD25 positive T-cells were separated at day 6 when their frequency showed at least a 10-fold increase above that observed among unstimulated PBMC. After magnetic sorting, purified CD25⁺ T cells were cultured in the presence of IL-2 alone without any restimulation. The number of cells obtained at day 25 using the CD25-sorting procedure was 4- to 5-fold greater than that of the cultures selected using the standard procedure (Fig. 8). For example, in the case of D5, about 10% of the stimulated parental line (6x10⁵ cells) were recovered by CD25 selection. This aliquot was amplified 100-fold in the presence of IL-2 without restimulation, reaching 60.10⁶ cells at day 25, while in the same time, the-culture obtained by the standard procedure showed no amplification. At day 30, each of the 3 CD25-selected lines was composed of at least 6.10⁷ cells while the 3 corresponding lines undergoing the standard procedure always represented much less than 4.10⁷ cells. All of the 11 cell lines derived by either the standard or CD25-selection protocols were cytotoxic for autologous BLCL but not for autologous PHA blasts, suggesting EBV-specific recognition.

### Example 5:

**Estimation of cell line purity in EBV-specific T cells (Table 2)**: To estimate the proportion of EBV-specific T cells within each T-cell line, T-cell clones were derived from bulk cultures by limiting dilution. Fifteen to twenty days after cloning, individual clones were split and tested for their ability to proliferate against autologous BLCL and each of two allogeneic BLCL. Due to the difficulty in finding fully mismatched BLCL for each donor, 2 control BLCL were used in each test to avoid false-positive results. In fact, this possibility seemed extremely rare since only 19 of the 640 T-cell clones tested (i.e < 3%) proliferated against all 3 of the target BLCL tested. Conversely, to avoid false-negative results, 26 of the clones negative against the 3 targets were reamplified and their absence of reactivity confirmed. Substantial variability was observed in cell line purity, ranging from 32% to 96%. To precisely compare the effect of CD25 positive selection on the resulting specific T-cell purity, type II or III cell lines were prepared and cloned in parallel from the same donors (D5 and D6). Specificity determination at the clonal level showed a dramatically increased frequency of autologous BLCL-specific T-cell clones in the CD25-selected lines D5^{III} and D6^{III} : 32 vs 96% and 61 vs 96% for D5^{II} vs D5^{III} and D6^{II} vs D6^{III}, respectively. In addition, immunoscope analysis revealed a decrease in diversity between these same populations. Taken together, these results indicate that early CD25 selection eliminated non-EBV specific T-cells which persisted or were amplified in cultures prepared using the standard selection procedure.

Among the 317 clones derived from the CD25-selected cultures, only 11 (3%) were unable to recognize the autologous BLCL. Moreover, it is tempting to speculate that these 3% non-specific T cells belonged to the few CD25⁺ cells already present among PBMC before stimulation that were detected among unstimulated PBMC, at least until day 6 after initiation of the culture. In this example, no CD25-depletion of PBMC was performed because the frequency of these antigen-specific cells was expected to be high.

To confirm that the panel of clones tested was representative of the bulk population analyzed, the authors of the invention compared the immunoscope profiles of the CD25-purified D5^{III} culture and the immunoscope profile obtained from the pool of 259 clones derived from it: approximately 60 clones were detectable in both cases, and most of the peaks were present in both analyses. Together with the estimated cloning efficiency, this provided further proof of the representativity of the clones tested.

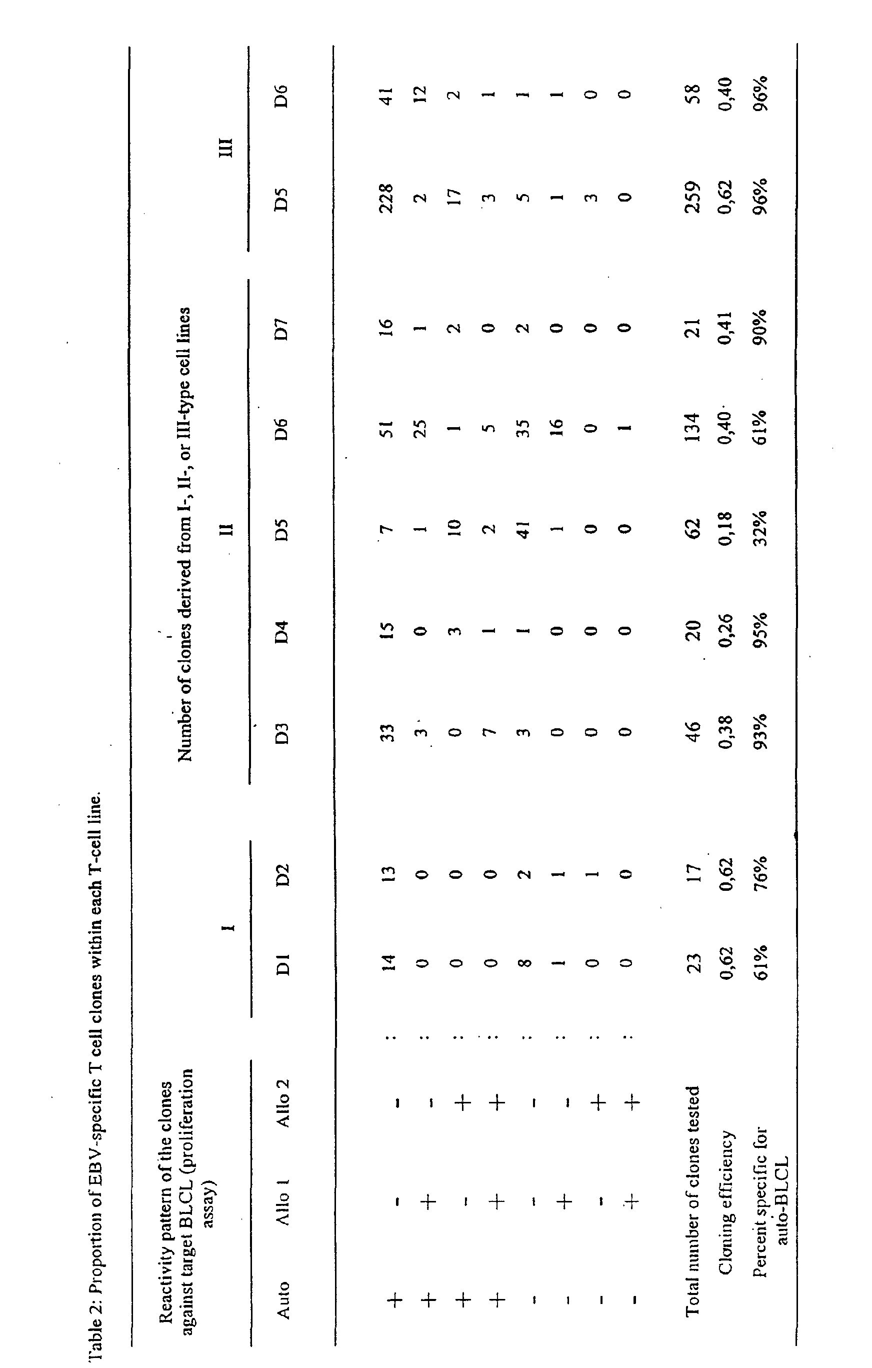

### Example 6:

**Analysis of anti-EBV T-cell responses in polyclonal cell lines:** To identify the EBV antigens recognized by the T-cell lines, a transient COS transfection assay was used allowing semiquantitative analysis of anti-EBV responses within polyclonal T-cell lines (Scotet et al, 1999). Decreasing numbers of responding polyclonal T cells (10⁵, 10⁴ and 10³) were incubated with COS cells transiently transfected with DNA coding for autologous class I HLA alleles and viral proteins, and the TNFα released by responding T cells was measured. The EBV proteins included in this analysis were the four well characterized EBV immediate early protein, BLF1 early proteins, BMLF1, BMRF1, BHRF1, the late protein BCRF1 and BRLF1, and the eight latent proteins (EBNA1, 2, 3A, 3B, 3C, LP, LMP1, LMP2). Thirty seven responses were observed (shown in Table 3): 7 against BZLF1 (in the context of HLA-B8, -B14, -B18, -B35 and Cw6); 7 against BMLF1 (in the context of HLA-A2 and - B18); 2 responses against BRLF1 (HLA-A2 and -B44) and 2 against BMRF1 (HLA-Cw6 and -B35). No response was observed against BHRF1 and BCRF1. Notably, 3 out of 3 HLA-B18 donors had a strong TNFα response against BZLF1 and 4 out of 5 HLA-A2+ donors showed a strong response against BMLF1 in this HLA context. Concerning the responses directed toward latent epitopes, 5 were detected against EBNA-3A, 2 weak responses against EBNA-3B, 6 against EBNA-3C and 6 against LMP2. Remarkably, 8 of the strongest responses detected at the bulk level were confirmed at the clonal level with a small panel of clones, consistent with a high frequency of clones having such specificity in the bulk culture. Taken together, these data show that the T-cell memory response reactivated against autologous BLCL is equally directed against EBV lytic (18 responses) and EBV latent proteins (19 responses). Finally, analysis of the D6^{II} and D6^{III} cell line specificities demonstrated that responses observed in the CD25-selected culture were the same as those observed in the control culture. Importantly, TNFα production by the CD25-selected population (D6^{III}) in response to EBV proteins was greater than that of the control population (D6^{II}). This finding is consistent with the enrichment in specific T-cells revealed by the structural analysis of T-cell line diversity (see above).

Six lines were selected using the standard protocol already used by others for clinical application (I- and II-type cell lines), and two lines were selected using the new method of the invention which required a single stimulation against autologous BLCL followed by a separation at day 6 of the CD25 positive activated T-cells (III-type cell lines).

The authors of the invention found that the EBV specific T-cell lines prepared using the standard protocol were composed of about 100 distinct T-cell clones. This number was decreased by 50% when the CD25 selection procedure was used. Thirty two to 95% of the T cell clones derived from type A and B cell lines were specific for the autologous BLCL, whereas 96% of the clones from lines obtained after CD25 selection were specific for the autologous BLCL. Concerning their specificity the authors of the invention demonstrated a high focusing of EBV recognition (32/37 of the specificities detected) toward 5 EBV proteins (BZLF1, BMLF1, EBNA-3A, EBNA-3C and LMP2).

The above results demonstrate that selection of the CD25+ activated T-cell fraction 6 days after a single specific stimulation had 4 main effects: 1) increasing the rate at which specific T cells are selected 2) retaining the specificities present in the culture prepared according to the conventional procedure 3) decreasing the overall diversity of the T cell-line and 4) increasing the frequency of EBV-specific T cell clones. Thus, this approach represents an improvement to the preparation of EBV-specific T cell lines for adoptive immunotherapy and should be considered for future clinical applications.

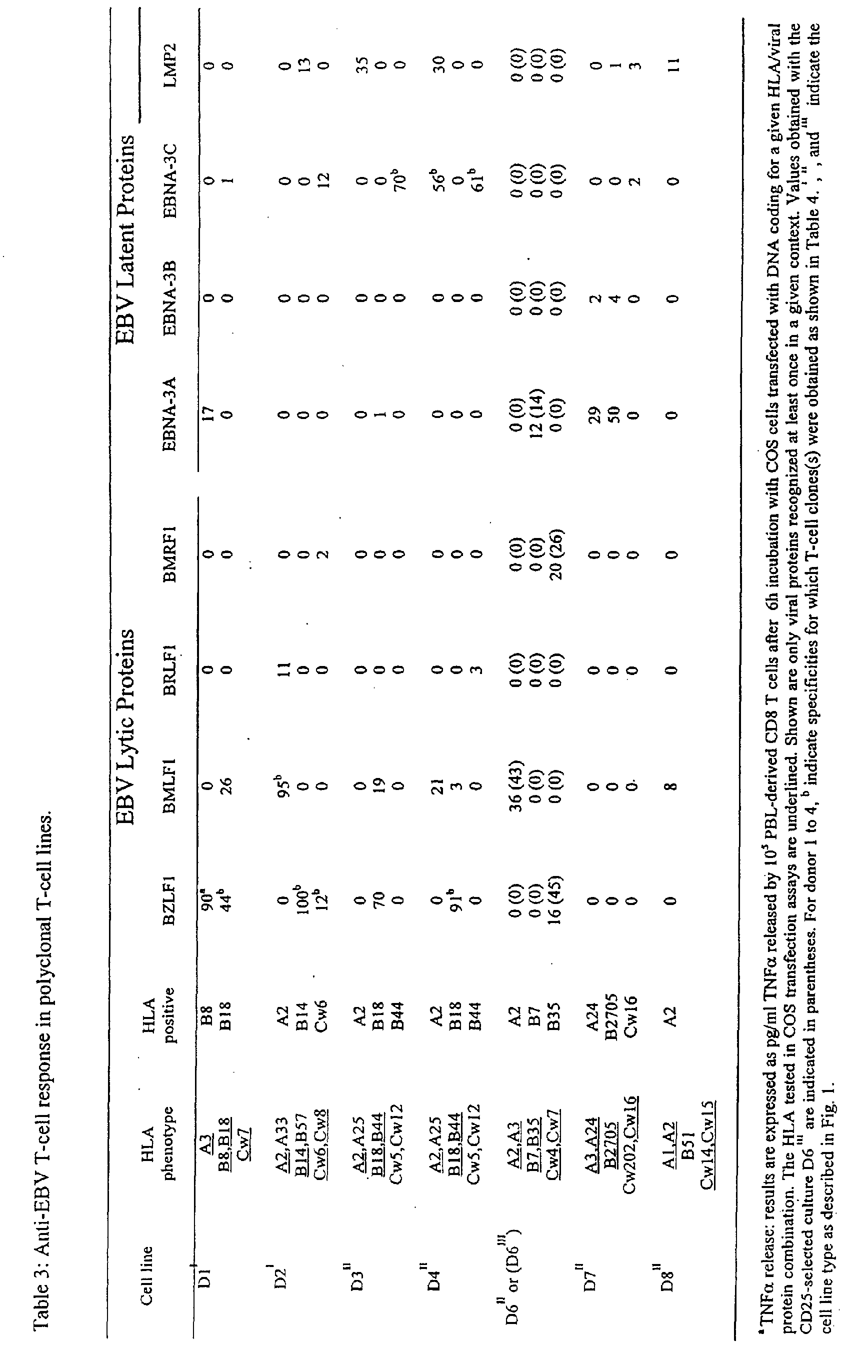

### Example 7

### Clonal analysis and epitope mapping (Table 4):

To precisely document the presence of CD4 specific T-lymphocytes among pp65 selected PBMC, CD4 T-cell clones were derived by limiting dilution from the bulk cultures of four different donors (n°8, 12, 15 and 20). After cloning, each clone was tested against the autologous BLCL loaded with one of the 50 peptides covering the entire pp65 sequence. Twenty seven out of the 28 CD4+ T-cell clones tested showed the pattern of reactivity presented strongly suggesting that this CD4+ population contained only one or a few specific distinct T-cell clones. Proliferation was observed against the autologous BLCL only when it was loaded with peptide n°4 and not with any other peptide from the panel. Further testing with 5 overlaping shorter peptides covering the sequence of peptide number 4, allowed identification of the minimal peptide L12Q. Finally, this recognition was abrogated in the presence of an HLA-DQ specific mAb (not shown) and was observed against HLA-DQ0602(+) target BLCL only ; thus demonstrating that HLA-DQ0602 was the restricting element. Using this approach CD4+ T-cell clones specific for peptide n°4, 14, 34 and 45 were identified from donor n°8, 12, 15 and 20 and CD8+ T cell clones specific for peptides n°25, 30 and 33 from donor 8 and 20. Table 4 summarizes the panel of pp65 epitopes identified during this study (SEQ ID NO: 1-9). In the case of donors 8 , 12 and 15 the testing of shorter peptides derived from the 23 mer initialy used for screening allowed identification of the minimal epitopes underlined in Table 4.

### Discussion

Different technologies may be considered for the enrichment of antigen-specific T lymphocytes. If the antigen is known already, i.e. the HLA-restricting molecule and the peptide presented, then soluble MHC tetramers, initially developed in the group of M. Davis (**24**) provide an elegant and powerful tool for the purification of antigen-specific T-cells. Indeed numerous recent seminal studies relied on this new technology. Another approach allowing detection and purification of live antigen-specific T-cells has been developed by the group of J. Scmitz in Germany (**25**). This latter method relies on the capability of memory /effector CD4+ (Th1-type) and CD8+ T-cells to secrete cytokines such as IFN_{γ} following a short-term antigenic restimulation with synthetic peptides. To purify INFγ secreting cells the authors developed a so-called affinity matrix technology which first consists in creating an affinity matrix for IFN_{γ} on the cell surface using Ab-Ab conjugates directed against CD45 and IFN_{γ} (anti-IFNγ-CD45). Then specific T-lymphocytes are allowed to secrete IFN_{γ} for a short period of time, which relocate on the Ab-Ab conjugates. Next, IFN_{γ} is stained with a PE-conjugated INF_{γ} specific Ab and finally magnetic activated cell sorting using anti-PE Ab microbeads can enrich PE-labeled cells. The efficiency of this procedure has been shown for Flu 58-66 peptide-specific IFN_{γ} producing T-cells and for recombinant tetanus toxoid Th2-type IL4-secreting CD4+ T-lymphocytes.

The tetramer technology is limited to T-cells with known specificities. The affinity matrix technology is limited to T cells that secrete a particular cytokine. This former limitation can become a significant concern if one wants to recover T cells from all components of a particular memory T cell repertoire. For example it has been recently demonstrated that immunological memory is displayed by distinct T cell subsets: CD45RA(-) CCR7(+) cells corresponding to lymph-node-homing cells lacking inflammatory and cytotoxic function (defined by the authors as central memory T cells TCM) and CD45RA(-) CCR7(-) cells corresponding to tissue-homing cells having various effector functions and in particular the ability to secrete INF_{γ}, IL-4 and IL-5. The authors defined these cells as effector memory T cells or TEM. Since different memory subsets display different cytokine profile this could render their global purification even more complicated using the affinity matrix technology.

For many years, non-specific stimulation procedures have been used to amplify T-lymphocytes. When optimal, such procedures allow amplification of all T-cells present in the culture and consequently do not affect their initial diversity. These methods rely on the use of large excess of autologous (when available) or allogeneic feeder cells made of PBMC, B lymphoblastoid cell line, a polyclonal T-cell activator such as PHA or an anti-CD3, and IL2. The growth rate of T-lymphocytes cultured under these conditions corresponds to a doubling time of between 24 and 35 hours.

The present invention now provides a novel method of clonal selection and amplification of activated T cells. The method is based, inter alia, on the negative and positive selection of CD25 cells. Surprisingly, although CD25-selection has been considered more than 10 years ago for the enrichment of T cells, to our knowledge no systematic approach for direct amplification of antigen specific T-cells using this principle has been developed so far and no method reported in the art shows that CD25 can be used to efficiently produce T cells for therapeutic purposes.

The present invention now discloses a clinically suitable strategy able to select in any genetic background the memory T-cell repertoire specific for a viral protein

The present invention demonstrates that virus specific memory T-cells can be purified through CD25-selection after direct stimulation of PBMC with a peptide, a mixture of peptide, and finally a viral vector encoding an entire protein. In particular, direct purification of pp65-specific CD8+ and CD4+ T cells after a single PBMC stimulation with a Canarypox viral vector encoding the entire pp65 protein strongly suggests that this method is probably bound to become the most straightforward approach to purify specific memory T-lymphocytes against a protein of interest irrespective of their genetic background. After ALVAC-pp65 stimulation, pp65 specific T-lymphocytes could be isolated from 6/11 of the CMV seropositive donors tested. Five out of these 6 donors presented both a CD8+ and a CD4+ positive response. Several reasons can account for the fact that pp65-specific T-lymphocytes were not isolated from all the donors tested. Although early studies have suggested that the human CTL response to CMV is dominated by CTL against pp65, the major immediate early protein (IE-1) has also been recognized as an important CTL target, thus, for some donors, the frequency of pp65 specific T-cells may have been too low to be isolated by our technique. In addition, Kern et al demonstrated that in some individuals CD8+ T cells recognized IE-1 but not pp65. Nevertheless we do not favor the latter explanation since in Kern's experience all donors nonresponsive to pp65 were HLA-A2 negatives, contrarily to all the donors tested in the present study.

Furthermore, the present invention also demonstrates the possibility to probe the T-cell repertoire for the presence of yet unknown specificities. While other strategies have been considered in the prior art to this end, such as intracellular cytokine staining and enzyme-linked immunospot (ELISPOT) assays for enumeration and characterization of antigen-specific CD4+ and CD8+ T cells, these methods cannot be used to purify live antigen-specific T-cells.

The present invention, based on a CD25 strategy, is not limited by a structural criterion (that is the MHC-peptide complex of the tetramer) nor by a specific functional status (that is the ability to secrete a particular cytokine). Moreover, anti-CD25 mAb are widely available compared to tetrameric complexes or the Ab-Ab conjugates required by the affinity matrix technology. Furthermore, the present invention allows the production of antigen-specific clones with high efficiency and high purity in a limited period of time, which is critical for clinical applications. In addition, in the context of allo-transplantation one can consider the possibility to use the same protocol but for negative selection in order to delete alloreactive T-cells from the sample before positive selection of viral Ag specific T cells.

According to previously published results, therapeutic doses of Ag-specific T cells are probably comprised between 10⁸ (4X10⁷ /ml for EBV), and several billions (in the case of CMV). In the example presented, 1,3x10⁸ pp65-selected T cells were obtained within 13 days with a purity of 64% as detected by INF_{γ} production starting with 10⁸ PBMC. It is believed that this would provide a sufficient virus specific T-cell repertoire to protect or cure a patient. Finally the new clinical possibilities offered by the strategy presented can be rapidly tested since its relies on methods and reagents, namely immunomagnetic sorting, canary pox-vector and anti-CD25 monoclonal antibodies, that have already been validated for clinical applications.

### REFERENCES

- Abimiku et al (1995) HIV-1 recombinant poxvirus vaccine induces cross-protection against HIV-2 challenge in rhesus macaques. Nat. Med. 1:331.
- Altman J. D., P. A. H. Moss, P. J. R. Goulder, D. H. Barouch, M. G. McHeyzer-Williams, J. I. Bell, A. J. McMichael, and M. M. Davis. 1996. Phenotypic analysis of antigen-specific T lymphocytes. Science 274: 94.
- Borst, J., and A. Cope. 1999. Turning the immune system on. Immunol Today. 20(4):156-158.
- Brosterhus, H., S. Brings, H. Leyendeckers, R. A. Manz, S. Miltenyi, A. Radbruch, M. Assenmacher, and J. Schmitz. 1999. Enrichment and detection of live antigen-specific CD4(+) and CD8(+) T cells based on cytokine secretion. Eur J Immunol. 29(12):4053-9.
- Burrows S. R., S. J. Rodda, A. Suhrbier, H. M. Geysen, and D. J. Moss. 1992. The specificity of recognition of a cytotoxic T lymphocyte epitope. Eur. J. Immunol. 22: 191.
- Cadoz et al (1992) Immunisation with canarypox virus expressing rabies glycoprotein. Lancet 339:1429.
- Ferrari et al (1997) Replication-defective canarypox (ALVAC) vectors effectively activate anti-human immunodeficiency virus-1 cytotoxic T lymphocytes present in infected patients: implication for antigen-specific immunotherapy. Blood 90:2406.
- Fleschhauer K., S. Tanzarella, H. J. Wallny, C. Bordignon, and C. Traversari 1996. Multiple HLA-A alleles can present an immunodominant peptide of the human melanoma antigen Melan-A/MART-1 to a peptide-specific HLA-A*0201+ cytotoxic T cell line. J. Immunol. 157: 787.
- Gaschet et al (1996) Acute graft versus host disease due to T lymphocytes recognizing a single HLA-DPB1*0501 mismatch. J. Clin. Invest. 98: 100.
- Heslop et al (1994) Clinical protocol:administration of neomycin resistance gene marked EBV specific CTL to recipients of mismatched-related or phenotypically similar unrelated donor marrow grafts. Human Gene Therapy 5: 381.
- Heslop et al, (1994) Donor T cells to treat EBV-associated lymphoma. N. Engl. J. Med. 331: 679.
- Lundin et al (1989) Positive selection of Tac- (CD25) positive cells following T-cell activation. Use of immunomagnetic separation and implications for T-cell cloning. J. Immunogen. 16: 185.
- Pannetier, C., M. Cochet, S. Darche, A. Casrouge, M. Zoller, and P. Kourilsky. 1993 (a). The sizes of the CDR3 hypervariable regions of the murine T-cell receptor beta chains vary as a function of the recombined germ-line segments. Proc Natl Acad Sci U S A. 90(9):4319-23.
- Pannetier, C., S. Delassus, S. Darche, C. Saucier, and P. Kourilsky. 1993 (b). Quantitative titration of nucleic acids by enzymatic amplification reactions run to saturation. Nucleic Acids Res. 21(3):577-83.
- Redchenko I. V. and Rickinson A. B.. 1999. Accessing Epstein-Barr virus-specific T-cell memory with peptide-loaded dendritic cells. J. Virol. 73: 334-342.
- Scotet, E., J. David-Ameline, M. A. Peyrat, A. Moreau-Aubry, D. Pinczon, A. Lim, J. Even, G. Semana, J. M. Berthelot, R. Breathnach, M. Bonneville, and E. Houssaint. 1996. T cell response to Epstein-Barr virus transactivators in chronic rheumatoid arthritis. Journal Of Experimental Medicine. 184(5):1791-800.
- Steven et al (1997) Immediate early and early lytic cycle proteins are frequent targets of the EBV-induced cytotoxic T cell response. J. Exp. Med. 185: 1605.
- Tartaglia J., O. Jarret, J. C. Neil, P. Desmettre, and E. pAoletti. 1993. Protection of cats against feline leukemia virus by vaccination with a canarypox recombinant, ALVAC-FL. J. Virol. 67:2370.
- Thiel, A., A. Scheffold, and A. Radbruch. 1998. Immunomagnetic cell sorting--pushing the limits. Immunotechnology. 4(2):89-96.
- Wakasugi H., J. Bertoglio, T. Tursz, and D. Fradelizi 1985. IL 2 receptor induction on human T lymhpocytes: role for IL 2 and monocytes. J. Immunol. 135: 321.
- Walter et al (1995) Reconstitution of cellular immunity against cytomegalovirus in recipients of allogeneic bone marrow by transfer of T cell clones from the donor. N. Engl. J. Med 333: 1038.
- Wills et al (1996) The human cytotoxic T-lymphocyte (CTL) response to cytomegalovirus is dominated by structural protein pp65: frequency, specificity, and T-cell receptor usage of pp65-specific CTL. J. Virol. 70: 7569.

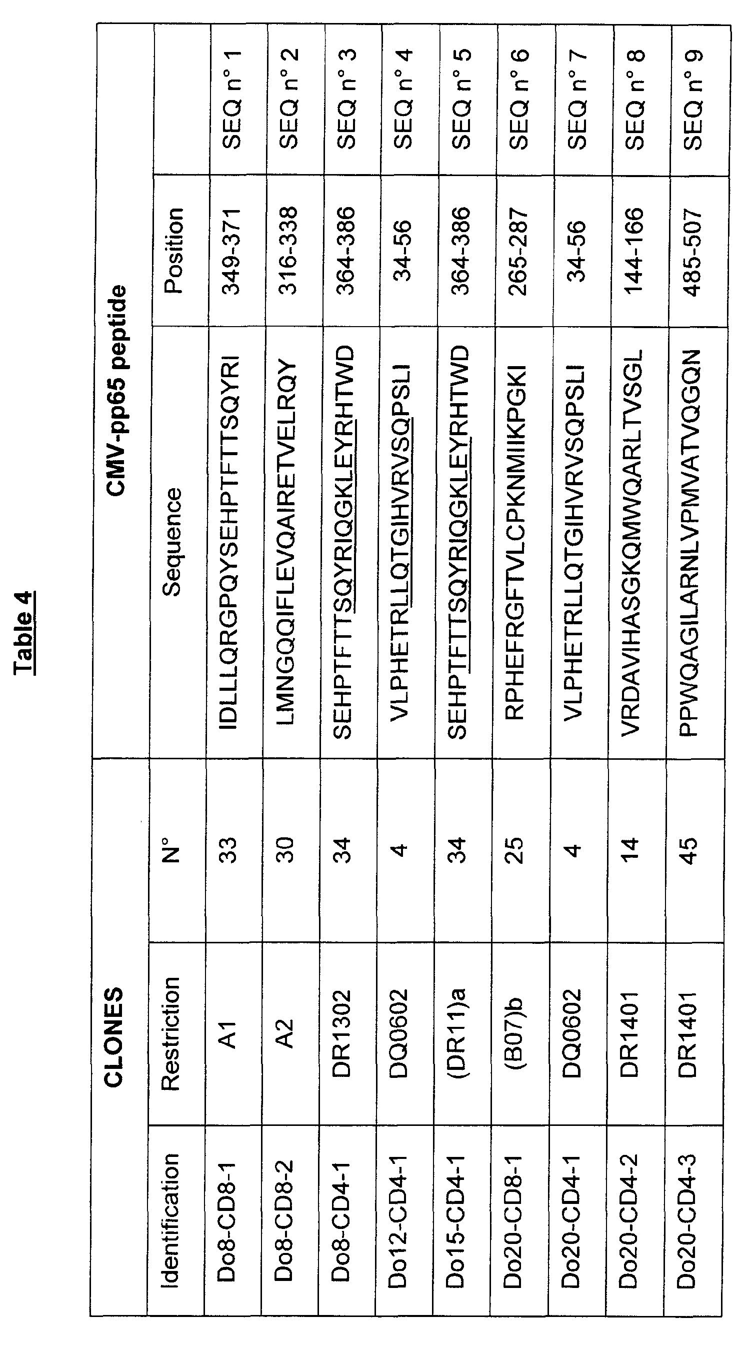

## Claims

1. A method for obtaining T-lymphocytes specific for known or unknown epitopes, the method comprising :
a. depleting CD25-positive cells present in a sample of peripheral blood mononuclear cells (PBMC);
b. Incubating the CD25-negative PBMC with an antigen likely to contain at least one epitope ;
c. Isolating the CD25-positive cells which have appeared ; and
d. Amplifying the isolated CD25-positive cells;
thereby obtaining T-lymphocytes specific for said epitope.

2. The method of claim 1, further comprising the step of conditioning the T lymphocytes in a pharmaceutically acceptable carrier or diluent.

3. The method according to claim 1, wherein said antigen is selected from the group consisting of a peptide, a mixture of peptide, a protein, a naturally occurring target cell, a target cell, a target cell transfected with a nucleotic vector coding for a peptide or a protein, a non-peptide antigen such as a hydrocarbon molecule, a cell infected by a virus or a bacterium, a tumor cell and fungi.

4. The method according to claim 3, wherein said antigen is selected from:
- a cell infected by the EBV virus,
- a mixture of overlapping peptides,
- a molecule which derives from a virus,
- a molecule which derives from a tumor cell,
- a tumor cell, and
- a cell transfected by a vector encoding a viral antigen or protein.

5. The method according to any of the preceding claims wherein said epitope is involved in the activation of the T-lymphocytes, particularly of the CD4 T-lymphocytes, called "helper" lymphocytes.

6. A method for identifying an unknown epitope wherein the specific T-lymphocytes isolated and amplified according to the method of claim 1 are further contacted with a fragment of said antigen, likely to contain said epitope, and the cytotoxicity, cytokine production or cell proliferation of said specific T-lymphocytes towards the said fragment of said antigen is assessed, these assays being repeated with each overlapping fragment of said antigen, whereby the epitopes fragment which triggers cytotoxicity, cytokine production or cell proliferation of the specific T-lymphocytes is identified.

7. The method according to claim 6 wherein said fragment of said antigen is presented to said specific T-lymphocytes via target cells which are loaded with said fragment.

8. A method for producing antigen-specific T lymphocytes in vitro, the method comprising :
a) obtaining a population of cells comprising T lymphocytes,
b) treating said population of cells to remove CD25-positive cells,
c) contacting the treated cell population with an antigen to effect a primo stimulation of T lymphocytes, and
d) isolating CD25-positive cells from the cells of step c), wherein said cells contain T lymphocytes specific for said antigen.

9. A method of preparation of a composition to stimulate an immune response in a subject, said composition comprising antigen-specific T lymphocytes, the method comprising:
a) treating a population of cells comprising T lymphocytes to remove CD25-positive cells,
b) contacting the treated cell population with an antigen to effect a stimulation of T lymphocytes,
c) isolating CD25-positive cells from the cells of step c), and
d) conditioning the cells in a pharmaceutically acceptable diluent or carrier.

10. The method of claim 9, wherein, prior to step a), the population of cells is contacted with peripheral blood mononuclear cells from the subject to remove alloreactive T cells from said population.

11. The method of claim 9, wherein the antigen is a viral antigen, preferably selected from an EBV or a CMV antigen.

12. The method of claim 11, wherein the antigen is all or an immunogenic fragment of EBV-early lytic protein BMLF1 or of CMV envelope phosphoprotein pp65.

13. The method of claim 11 or 12, wherein the antigen is presented by an antigen-presenting cell.

14. A method of stimulating an antigen-specific immune response in an immunodeficient subject, the method comprising :
a) treating a population of cells comprising T lymphocytes to remove CD25-positive cells,
b) contacting the treated cell population with an antigen to effect a stimulation of T lymphocytes,
c) isolating CD25-positive cells from the cells of step c),
d) optionally, expanding the population of CD25-positive cells by in vitro culture,
e) conditioning the cells in a pharmaceutically acceptable diluent or carrier, and
f) injecting the population of CD25-positive cells to the subject.

15. A method of preventing or reducing viral infection in a subject during or after bone marrow transplantation, the method comprising injecting to a subject at risk of developing viral infection a composition comprising T cells specific for a virus, said composition being obtained by:
a) treating a population of cells comprising T lymphocytes from a donor subject, to remove CD25-positive cells,
b) contacting the treated cell population with a viral antigen to effect a stimulation of T lymphocytes,
c) isolating CD25-positive cells from the cells of step c),
d) optionally, expanding the population of CD25-positive cells by in vitro culture, and
e) conditioning the cells in a pharmaceutically acceptable diluent or carrier.

16. A peptide comprising a T cell epitope, wherein the peptide has a sequence of an epitope prepared or identified by the method of claim 6.

17. A peptide selected from SEQ ID NO: 1-4 and 7-9 or an immunogenic fragment thereof.


SEQUENCE LISTING

<110> INSERM

<120> Method for obtaining specific T-lymphocytes, and for
      identifying unknown epitopes.

<130> B0070EP1

<140>
<141>

<160> 11

<170> PatentIn Ver. 2.1

<210> 1
<211> 24
<212> PRT
<213> Artificial Sequence

<220>
<223> Description of Artificial Sequence: CMV-pp65
      peptide

<400> 1
Ile Asp Leu Leu Leu Gln Arg Gly Pro Gln Tyr Ser Glu His Pro Thr
  1               5                  10                  15

Phe Thr Thr Ser Gln Tyr Arg Ile
             20

<210> 2
<211> 23
<212> PRT
<213> Artificial Sequence

<220>
<223> Description of Artificial Sequence: CMV-pp65
      peptide

<400> 2
Leu Met Asn Gly Gln Gln Ile Phe Leu Glu Val Gln Ala Ile Arg Glu
  1               5                  10                  15

Thr Val Glu Leu Arg Gln Tyr
             20

<210> 3
<211> 24
<212> PRT
<213> Artificial Sequence

<220>
<223> Description of Artificial Sequence: CMV-pp65
      peptide

<400> 3
Ser Glu His Pro Thr Phe Thr Thr Ser Gln Tyr Arg Ile Gln Gly Lys
 1               5                  10                  15

Leu Glu Tyr Arg His Thr Trp Asp
            20

<210> 4
<211> 23
<212> PRT
<213> Artificial Sequence

<220>
<223> Description of Artificial Sequence: CMV-pp65
      peptide

<400> 4
Val Leu Pro His Glu Thr Arg Leu Leu Gln Thr Gly Ile His Val Arg
 1               5                  10                  15

Val Ser Gln Pro Ser Leu Ile
            20

<210> 5
<211> 24
<212> PRT
<213> Artificial Sequence

<220>
<223> Description of Artificial Sequence: CMV-pp65
      peptide

<400> 5
Ser Glu His Pro Thr Phe Thr Thr Ser Gln Tyr Arg Ile Gln Gly Lys
 1               5                  10                  15

Leu Glu Tyr Arg His Thr Trp Asp
            20

<210> 6
<211> 23
<212> PRT
<213> Artificial Sequence

<220>
<223> Description of Artificial Sequence: CMV-pp65
      peptide

<400> 6
Arg Pro His Glu Phe Arg Gly Phe Thr Val Leu Cys Pro Lys Asn Met
  1               5                  10                  15

Ile Ile Lys Pro Gly Lys Ile
            20

<210> 7
<211> 23
<212> PRT
<213> Artificial Sequence

<220>
<223> Description of Artificial Sequence: CMV-pp65
      peptide

<400> 7
Val Leu Pro His Glu Thr Arg Leu Leu Gln Thr Gly Ile His Val Arg
  1               5                  10                  15

Val Ser Gln Pro Ser Leu Ile
            20

<210> 8
<211> 23
<212> PRT
<213> Artificial Sequence

<220>
<223> Description of Artificial Sequence: CMV-pp65
      peptide

<400> 8
Val Arg Asp Ala Val Ile His Ala Ser Gly Lys Gln Met Trp Gln Ala
1               5                   10                  15

Arg Leu Thr Val Ser Gly Leu
            20

<210> 9
<211> 23
<212> PRT
<213> Artificial Sequence

<220>
<223> Description of Artificial Sequence: CMV-pp65
      peptide

<400> 9
Pro Pro Trp Gln Ala Gly Ile Leu Ala Arg Asn Leu Val Pro Met Val
1               5                   10                  15

Ala Thr Val Gln Gly Gln Asn
            20

<210> 10
<211> 9
<212> PRT
<213> Artificial Sequence

<220>
<223> Description of Artificial Sequence: peptide from
      the CMV-enveloppe phosphoprotein pp65

<400> 10
Asn Leu Val Pro Met Val Ala Thr Val
1               5

<210> 11
<211> 9
<212> PRT
<213> Artificial Sequence

<220>
<223> Description of Artificial Sequence: Peptide from
      the EBV-early lytic proteine BMLF1

<400> 11
Gly Leu Cys Thr Leu Val Ala Met Leu
1               5